# EUROPEAN PATENT APPLICATION

(11) **EP 1 878 393 A1**
(43) Date of publication of application: **16.01.2008**
(21) Application number: 06014273.4
(22) Date of filing: 10.07.2006
(51) Int. Cl.: A61B 17/17

(54) **Drill assembly for bone reconstruction plate**

(71) Applicant: Stryker Trauma GmbH, 24232 Schönkirchen/Kiel (DE)
(72) Inventor: Giersch, Helge, 24103 Kiel (DE); Prien, Ole, 24145 Kiel (DE); Cremer, Axel, 23759 Fahrenkrug (DE); Seemann, Michael, 24145 Kiel (DE)
(74) Representative: Dilg, Andreas

(57) **Abstract**

A drill assembly for a bone reconstruction plate includes an aiming arm, an outer guide, and a drill guide. The aiming arm includes an alignment bore which defines an alignment bore center line. The outer guide is operable for insertion into the alignment bore, the outer guide including an outer guide bore extending therethrough. The drill guide is operable for insertion into the outer guide bore of the outer guide, the drill guide including a drill guide bore for receiving a drill bit, the drill guide bore defining a drill guide bore center line which is offset from the alignment bore center line.

## Description

### BACKGROUND

The present invention relates to drill assemblies for bone reconstruction plates.

A drill assembly for a bone reconstruction plate provides a platform for accurately locating and drilling screw holes for attachment screws that are used to secure the bone reconstruction plate to the injured bone. In a typical configuration, the assembly includes an aiming arm positioned over the bone reconstruction plate, the aiming arm having several alignment bores for receiving and guiding a drill guide to the center of the screw holes in the bone reconstruction plate. In a typical implementation, screws are used to secure the drill guide to the aiming arm, such that the drill guide does not move during drilling operations or movement of the assembly.

While the conventional approach is generally effectively, it is lengthy due to the amount of time needed to secure the drill assembly to the aiming arm. The time needed to install and remove the drill guide securing screws extends the time of the operation, and a more time efficient technique is needed to reduce the procedure time. Further disadvantageously, the conventional drill assembly does not readily accommodate for mis-alignments which can occur between the alignment bores in the aiming arm and the screw holes in the bone reconstruction plate, e.g., in areas where the bone plate has been shaped or bent to conform to the contours of a bone surface. In such circumstances, adjustment of one or both structures is needed to provide correct drill placement, further extending the procedure time of the operation. A capability is accordingly needed, whereby the position of the drill guide can be easily adjusted to correctly place the drill in the desired position.

### SUMMARY

The present invention provides a drill assembly for a bone reconstruction plate which can be securely, but quickly removed from the aiming arm, thereby reducing procedure time for the operation. The present invention further enables adjustment of the drill guide's position within the alignment bore, permitting placement of the drill bit in the desire location within the screw hole of the bone reconstruction plate.

In one embodiment of the invention, the drill assembly for a bone reconstruction plate includes an outer guide, and a drill guide. The outer guide is operable for insertion into an alignment bore of an aiming arm, and includes an outer barrel, a securing member disposed on the outer surface of the outer barrel, and an outer guide bore which extends through the outer barrel and the securing member. The drill guide is operable for insertion into the outer guide bore, and includes a drill guide barrel, an engagement member disposed on an outer surface of the drill guide barrel, and a drill guide bore which extends through the drill guide barrel. The engagement member disposed on the outer surface of the drill guide barrel is operable to provide a press-compression fit within the securing member of the outer guide to thereby secure the drill guide to the outer guide.

In another embodiment of the invention, the drill assembly for a bone reconstruction plate includes an aiming arm, an outer guide, and a drill guide. The aiming arm includes an alignment bore which defines an alignment bore center line. The outer guide is operable for insertion into the alignment bore, the outer guide including an outer guide bore extending therethrough. The drill guide is operable for insertion into the outer guide bore of the outer guide, the drill guide including a drill guide bore for receiving a drill bit, the drill guide bore defining a drill guide bore center line which is offset from the alignment bore center line.

These and other features of the invention will be better understood in view of the drawing and detailed description present below.

### BRIEF DESCRIPTION OF THE DRAWINGS

Fig. 1 illustrates an exemplary bone reconstruction plate drill assembly in accordance with the present invention.
Fig. 2A illustrates a detailed embodiment of the bone reconstruction plate drill assembly shown in Fig. 1 in accordance with the present invention.
Fig. 2B illustrates a cross-sectional view of the bone reconstruction plate drill assembly of Fig. 1 in accordance with the present invention.
Fig. 3A illustrates an outer guide configured in accordance with one embodiment of the present invention.
Fig. 3B illustrates an outer guide in two positions according to one embodiment of the present invention.

For clarity, previously described feature retain their reference numerals in subsequent drawings.

### DETAILED DESCRIPTION OF EXEMPLARY EMBODIMENTS

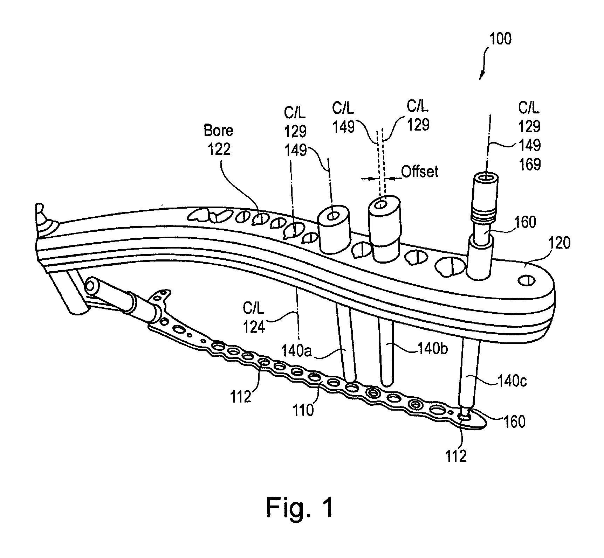

Fig. 1 illustrates an exemplary bone reconstruction plate drill assembly in accordance with the present invention. As shown, the assembly 100 includes a bone reconstruction plate 110, a radiolucent aiming arm 120, an outer guide 140 (three exemplary embodiments 140a, 140b, and 140c shown), and a drill guide 160. The bone reconstruction plate 110 is adapted for attachment to a bone, and includes one or more holes 112 for receiving screws, or other attachment means operable to secure the plate 110 to a bone. The opening of the screw holes 112 may be generally circular in shape, or they may be non-circular, e.g., elongated/elliptical in shape. The bone reconstruction plate 110 may be formed from a variety of materials, such as surgical stainless steel, Ti, or other suitable metals or materials.

The aiming arm 120 includes one or more alignment bores 122 for guiding the drilling apparatus into the desired target location within a screw hole 112. Each of the alignment bores 122 defines a center line 129 which is substantially aligned with (i.e., on boresight with) the center of the screw holes 112. The aiming arm 120 may be constructed from an X-ray transparent type of material, for example, injection molded thermoplastic containing, e.g., 70 % poly Ether Ether Ketone (PEEK) and 30% carbon fibers.

The assembly 100 further includes an outer guide 140 operable for insertion into an alignment bore 122, and a drill guide 160 operable for insertion into the outer guide 140. The outer guide and drill guide 160 operate to position the drilling apparatus to the desired target drilling location within a screw hole 112.

Three exemplary outer guides 140a, 140b, and 140c are shown. In the first example 140a, the center line of the outer guide 149 is aligned with the center line of the alignment bore 129. In the second example, the outer guide center line 149 is offset from the center line of the alignment bore 129. Aligning the outer guide center line 149 offset from the alignment bore center line 129 may be advantageous to correct mis-alignment between the bone reconstruction plate 110 and the aiming arm 120, whereby the offset alignment places the drilling target on boresight within the desired screw hole 112. In another embodiment, when the alignment bore center line 129 is on-boresight with the screw hole, aligning the outer guide center line 149 offset from the alignment bore center line 129 may also be advantageous in order to provide a more secure attachment bone plate attachment point. The third example of the outer guide 140c illustrates mutual alignment of the alignment bore, outer guide and drilling bore center lines 129, 149 and 169. The specific construction of the outer guide 140 is further described below.

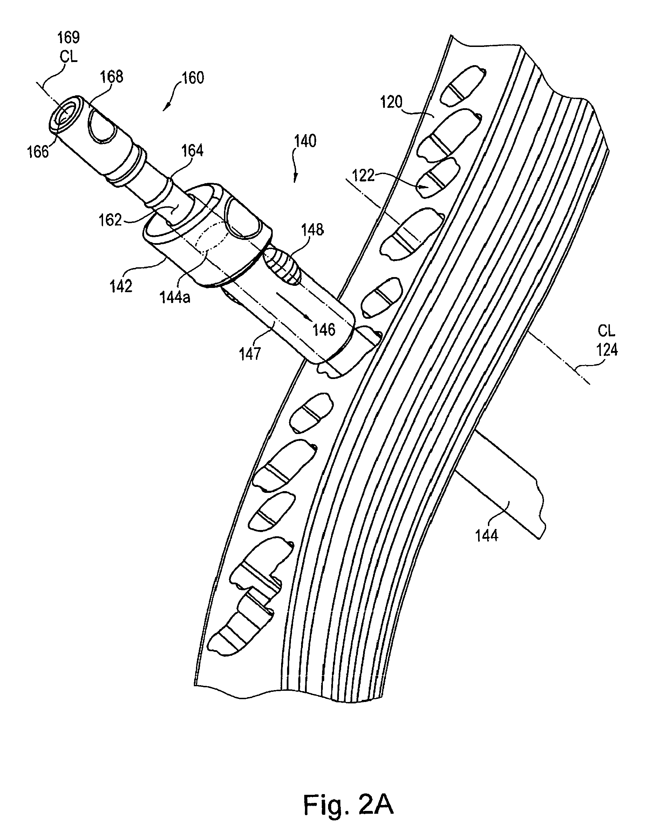

Fig. 2A illustrates a detailed embodiment of the drill alignment assembly shown in Fig. 1 in accordance with the present invention, with previously identified features retaining their reference numerals. The outer guide 140 is shown inserted into an alignment bore 122, and a drill guide 160 inserted into the outer guide 140. The outer guide 140 includes a securing top 142 which is formed on an outer barrel 144, whereby an outer guide bore 146 extends through each of the securing top 142 and the outer barrel 144. The outer guide 140 further includes an engagement sleeve 147, which in a particular embodiment has an outer diameter which is substantially that of the hole diameter of the alignment bore 122 into which the outer guide 140 is to be press-fit. In such an embodiment, the outer diameter of the engagement sleeve 147 defines substantially the alignment bore center line 129. The outer guide 140 further includes one or more locking members 148 operable to provide a press-compression fit within the alignment bore 122.

The drill guide 160 is operable for insertion into the outer guide bore 146, and includes a drill guide barrel 162, and an engagement member 164 disposed on the drill guide barrel's outer surface. A drill guide bore 166 for receiving a drill bit extends through the drill guide barrel 162 along a center line 169. In particular, the engagement member 164 is operable to provide a press compression fit within the securing top 142 of the outer guide to thereby secure the drill guide 160 to the outer guide 140. In this manner, the assembly can be moved and turned upside down without the drill guide 160 disengaging from the outer guide 140. Drill guide grip 168 facilitates removal of the drill guide 160 from the outer guide 140.

In a particular embodiment, the securing top 142 is formed from an elastic material, such as rubber or similarly resilient materials. It will be understood that such materials will be able to provide a press compression fit operable to hold the drill guide 160 into the outer guide 140 during movement of the assembly, while enabling the drill guide's removal from the outer guide 160 with only a small increase in pulling force.

The outer guide 140 may further include an engagement member stop 144a which is operable to inhibit further travel or insertion of the drill guide 160 into the outer guide 140. Further travel of the drill guide 160 into the outer guide 140 could result in the drill guide barrel 162 forcefully impacting the bone reconstruction plate 110, resulting in mis-alignment of the drilling process, or damage to the drill guide barrel 162 and/or bone reconstruction plate 110. In a specific embodiment, the engagement member 164 is formed as a raised annular ring extending around the outer surface of the drill guide barrel 162, and the engagement member stop 144a provides a diameter which is greater than the outer diameter of the drill guide barrel 162 to permit passage of the outer surface therethrough, but which is smaller than the clearance needed for the raised annular ring 164. This arrangement will prevent further travel of the drill guide 160 into the outer guide 140.

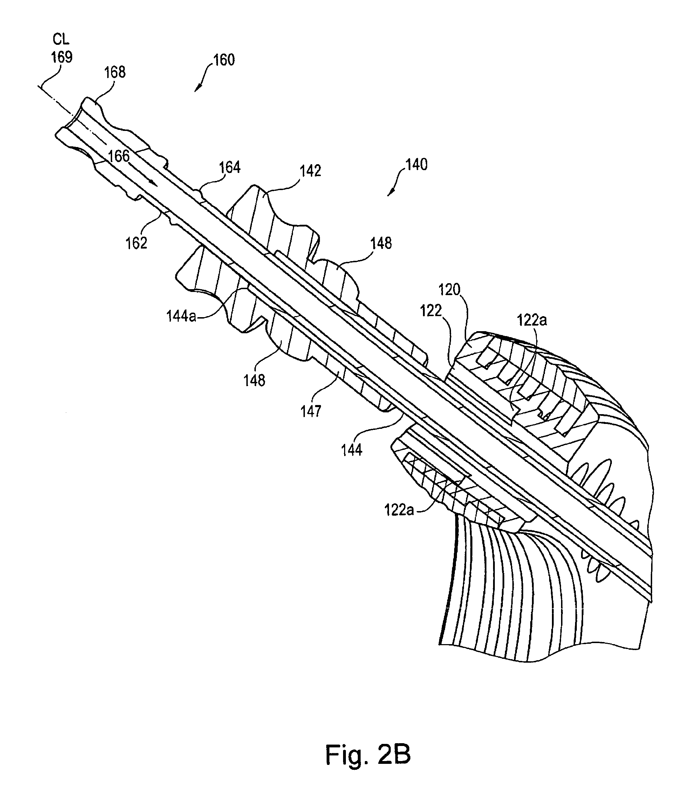

Fig. 2B illustrates a cross-sectional view of the drill assembly 100 in accordance with the present invention, with previously identified features retaining their reference numerals. As illustrated, the outer guide 140 is formed so as to make a press compression fit into a bore 122 of the aiming arm 120. One or more resilient locking members 148 are formed onto the engagement sleeve 147 of the outer guide 140, the locking members 148 formed generally complementary to a cut-out pattern in the bores 122 of the aiming arm 120, the engagement sleeve 147 and the locking members 148 composed of a resilient elastic material. Upon insertion of the outer guide 140 into an alignment bore 122, the engagement sleeve 147 and the resilient locking members 148 become depressed to form a press compression fit within the bore 122, thereby retaining the outer guide 140 therein. A locking member stop 122a disposed within the alignment bore 122 (shown as a counter-sunk diameter bore included within alignment block bore 122) is used to stop further travel of the engagement sleeve 147 and the locking members 148 into the alignment bore 122. Removal of the outer guide 140 from the alignment block 122 is effectuated by supply sufficient force (e.g. upward force) to overcome the compression fit. Each of the outer guide 140 and the drill guide 160 may include grips formed at a convenient location of their respective structures.

As noted above and illustrated in Fig. 1, the drill guide bore 166 and the alignment bore 122 may be centered with respect to each other (i.e., center lines 129 and 169 are mutually aligned), or alternatively, arranged in an offset manner. Alignment of the drill guide and alignment bore center lines 129 and 169 is advantageous when the alignment bore is substantially on-boresight with the center of the screw hole 112. However, some mis-alignment between the screw hole 112 and the aiming arm 120 can occur, in which case the center line 124 may not be sufficiently on boresight of the screw hole 112. In this case, an adjustment of the drill guide 160 to provide a matching offset is needed in order to place the drill guide center line 169 on boresight with the mis-aligned screw hole.

In another embodiment, the drill guide center line 169 may be sufficiently on-boresight with the screw hole 112, however, offset placement of the drilled hole from the screw hole is desired in order to provide a more advantageous screw position. Such an offset placement may be needed in order to provide an attachment point through a bent portion of the bone reconstruction plate 110. As noted above, the screw holes 112 can be made elongated or elliptical in their opening to accommodate different screw positions.

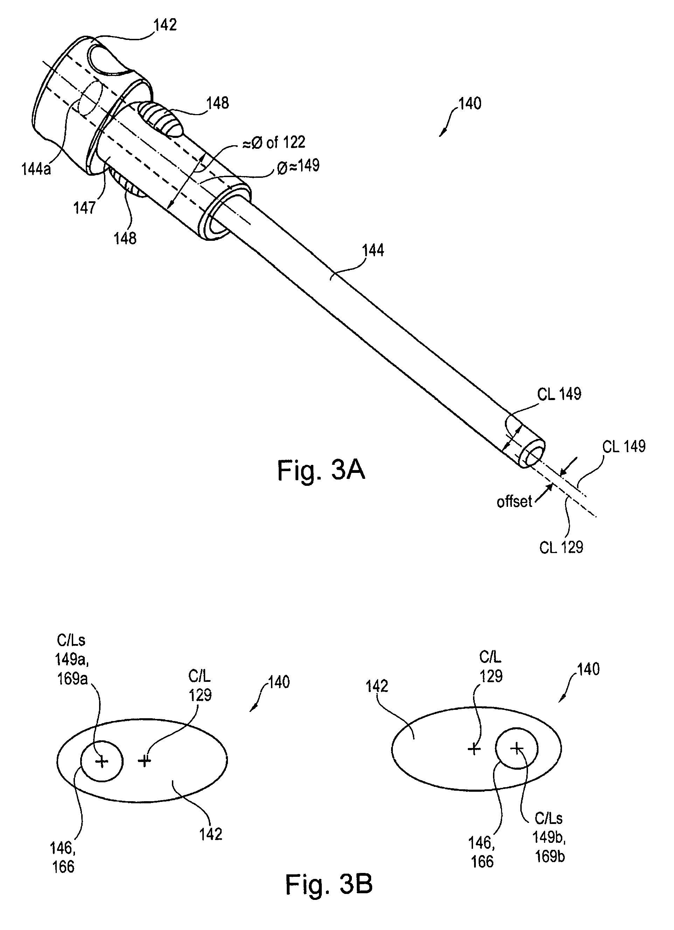

Fig. 3A illustrates an outer guide 140 configured in accordance with one embodiment of the present invention. As shown, the outer guide 140 is operable to provide a center line 149 which is offset from the center line 129 of the alignment bore. The outer guide 140 includes an engagement sleeve 147 having an outer diameter which is substantially that of the hole diameter of the alignment bore 122 into which the outer guide 140 is to be press-fit. Accordingly, the outer diameter of the engagement sleeve 147 defines substantially the center line 129, the center line 129 of the alignment bore 122 being substantially aligned with the center line of the screw hole 112.

The outer guide 140 further includes a bore 146 which extends through the securing member 142 and the outer guide barrel 144, defining a center line 149. The outer guide bore 146 is operable to receive the drill guide 160, the construction of the drill guide 160 being substantially as described above. When inserted into the outer guide 140, the drill guide 160 will have a center line substantially that of the center line 149. Accordingly, the center line 169 of the drill guide 160 will be offset from the center line of the screw hole 112. In this manner, securely aligning the drill guide 160 to drill holes for an offset screw can be accomplished.

In an extension of the foregoing, the outer guide 140 can be rotated 180 degrees to provide a center line 149 on the opposite side of the alignment bore center line 129. Fig. 3B illustrates this arrangement in which an outer guide 140 is shown in two positions, one rotated 180 degrees relative to the other. The left most illustration shows the outer guide 140 having a center line 149a disposed to the left side of the alignment bore center line 129, and the right most illustration shows the outer guide 140 having a center line 149b disposed to the right side of the alignment bore 129. The outer guide bores 146 are operable to receive a drill guide 160, the drill guides 160 having respective drill guide bores 166 defining center lines 169a and 169b which are substantially that of the outer guide bore center lines 149a and 149b. Accordingly, a screw hole which is offset in either the left or right side of the alignment bore center line 129 can be correctly aligned to. In addition, if the aiming arm 120 and the outer guide 140 are appropriately configured to provide four different engaging positions (e.g., the aiming arm having four cut-outs within bore 122, and the outer guide 140 having four locking members 148), the outer guide 140 can be rotated 90 degrees to provide offset alignments in four different positions around the alignment bore center line 129. Of course, the aforementioned technique can be extended to provide a larger number of offset positions around the alignment bore center line 129.

The foregoing description has been presented for purposes of illustration and description. It is not intended to be exhaustive or to limit the invention to the precise form disclosed, and obviously many modifications and variations are possible in light of the disclosed teaching. The described embodiments were chosen in order to best explain the principles of the invention and its practical application to thereby enable others skilled in the art to best utilize the invention in various embodiments and with various modifications as are suited to the particular use contemplated. It is intended that the scope of the invention be defined solely by the claims appended hereto.

## Claims

1. A drill assembly for a bone reconstruction plate, the drill assembly comprising:
an outer guide (140) operable for insertion into an alignment bore (122) of an aiming arm (120), the outer guide (140) comprising a securing member (142) disposed on an outer barrel (144), wherein a guide bore (146) extends through each of the securing member (142) and the outer barrel (144); and
a drill guide (160) operable for insertion into the outer guide bore (146), the drill guide (160) comprising a barrel (162) and an engagement member (164) disposed on an outer surface of the drill guide barrel (162), wherein a drilling bore (166) for receiving a drill bit extends through the drill guide barrel (162),
wherein the engagement member (164) disposed on the outer surface of the drill guide barrel (162) is operable to provide a press-compression fit within the securing member (142) of the outer guide to thereby secure the drill guide (160) to the outer guide (140).

2. The drill alignment assembly of claim 1, wherein the outer guide (140) further includes an engagement member stop (144a) operable to prevent advancement of the engagement member therebeyond, thereby inhibiting further insertion of the drill guide (160) into the outer guide (140).

3. The drill alignment assembly of claims 1 or 2, wherein the securing member (142) comprises an elastic material.

4. The drill alignment assembly of any one of claims 1-3, wherein the drill guide bore (166) comprises a center line (169) which is substantially aligned with a center line (129) of the alignment bore (122).

5. The drill alignment assembly of any of claims 1-5, wherein the drill guide bore (166) comprises a center line (169) which is offset from a center line (129) of the alignment block bore (120).

6. The drill assembly of claim 5, wherein the drill guide bore (166) comprises a first offset center line 169a when the outer guide (140) is in a first position within the alignment bore (122), and wherein the drill guide bore (166) comprises a second offset center line (169b) when the outer guide (140) is in a second position within the alignment bore (122).

7. A drill assembly for a bone reconstruction plate, the drill alignment assembly comprising:
an aiming arm (120) operable to position a drilling target within a screw hole (112) of a bone reconstruction plate (110), the aiming arm (120) comprising an alignment bore (122) which defines an alignment bore center line (129);
an outer guide (140) operable for insertion into the alignment bore (122), the outer guide (140) comprising a guide bore (146); and
a drill guide (160) operable for insertion into the outer guide bore (146), the drill guide (160) comprising a drilling bore (166) for receiving a drill bit, the drilling bore (166) defining a drill guide center line (169),
wherein the drill guide center line (169) is offset from the alignment bore center line (129).

8. The drill assembly of claim 7, wherein the drill guide bore (166) comprises a first offset center line 169a when the outer guide (140) is in a first position within the alignment bore (122), and wherein the drill guide bore (166) comprises a second offset center line (169b) when the outer guide (140) is in a second position within the alignment bore (122).

9. The drill alignment assembly of claims 7 or 8,
wherein the outer guide (140) comprises a securing member (142);
wherein the drill guide (160) comprises an engagement member (164); and
wherein the engagement member (164) is operable to provide a press compression fit within the securing member (142) of the outer guide (140) to thereby secure the drill guide (160) to the outer guide (140).

10. The drill alignment assembly of any one claims 7-9, wherein the outer guide (140) further includes an engagement member stop (144a) operable to prevent advancement of the engagement member (144) therebeyond, thereby inhibiting further insertion of the drill guide (160) into the outer guide (140).
